# EUROPEAN PATENT APPLICATION

(11) **EP 1 005 831 A2**
(43) Date of publication of application: **07.06.2000**
(21) Application number: 99123939.3
(22) Date of filing: 03.12.1999
(51) Int. Cl.: A61B 5/103

(54) **A method for measurement of a penile diameter**

(30) Priority: 04.12.1998 JP 34579598
(71) Applicant: Eisai Co., Ltd., Tokyo (JP)
(72) Inventor: Adachi, Hideyuki, Inashiki-gun, Ibaraki (JP)
(74) Representative: HOFFMANN - EITLE

(57) **Abstract**

The object of the invention is to establish a measurement method wherein the increase in penile diameter can be monitored for long periods, without the damage to tissues by insertion of a measurement needle as the disadvantage of conventional methods measuring the internal pressure of the corpus cavernosum. According to the invention, a pair of piezoelectric crystals are fitted on the opposite surfaces of the adventitia of the penile erectile chamber using an adhesive agent and connected via an ultrasonic dimension system to a polygraph system, and the increase in the penile diameter upon erectile response is continuously recorded on a chart in a recorder.

## Description

### Field of the Invention

The present invention relates to a method for measurement of the penile diameter of an animal, which is useful for development of a therapeutic agent for erectile dysfunction.

### Prior Art

According to the report "the Massachusetts Male Aging Study" (J. Urol., 151, 54-61, 1994) published in 1994, the total number of 40- to 70-year-old potential patients with male erectile dysfunction is calculated at 18 millions and it is therefore concluded that male erectile dysfunction should be dealt with as a great problem in health. There is demand for higher standards of living with the advent of an aging society in Japan too, where male erectile dysfunction is estimated to be not only a medical problem but also a social problem in the future.

To treat male erectile dysfunction, intracavernosal injection, transurethral suppository, vacuum erection devices and penile prosthesis are practically used at present. Furthermore, sildenafil as a phosphodiesterase 5 (PDE 5) inhibitor developed by Pfizer came to be clinically usable recently in the US, European countries and Japan.

The measurement of intracavernous pressure in rats (J. Urol., 149, 627-632, 1993; Br. J. Pharmacol., 108, 1038-1042, 1993), rabbits (Acta Physiol. Scand., 143, 299-304, 1991) and dogs (Urology, 14, 347-352, 1984; Am. J. Physiol., 264, H419-H422, 1993) has been used mainly as a preclinical method for evaluating penile erectile function in an in vivo model. The disadvantage of these existent methods is that because of damage to the corpus cavernosum by insertion of needle, bleeding at the insertion site and coagulation in the tube measuring intracavernous pressure, it is not possible to monitor the intracavernous pressure for a long time.

### Disclosure of the Invention

The object of the present invention is to establish an evaluation method wherein the increase in penile diameter induced by electrical stimulation of the cavernous nerve can be monitored for long periods without any damage to the corpus cavernosum as a disadvantage of the existent measurement methods.

As a result of their eager study, the present inventor established a novel evaluation method wherein the increase in penile diameter induced by electrical stimulation of the cavernous nerve can, without damaging the corpus cavernosum, be monitored for long periods by measuring the penile diameter by fitting piezoelectric crystals on the outer diameter of the penis, and the present invention was thereby completed.

In the present invention, a pair of piezoelectric crystals are fitted using a medical adhesive respectively on the opposite parts of the outside of an animal penis, then signals from the piezoelectric crystals are received by an ultrasonic dimension system, and the change in penile diameter by electrical stimulation, by a drug or both by a drug and electrical stimulation is continuously recorded on a chart in a recorder via a polygraph system connected to an ultrasonic dimension system.

The invention is a method of measuring a penile diameter of an animal by use of piezoelectric crystals, being preferably applied to an animal such as dog, rabbit, rat and mouse.

It is optional that signals from a pair of piezoelectric crystals fitted on the opposite surfaces of the adventitia of the penile erectile chamber are received by an ultrasonic dimension system, and the diameter of the animal's penis is continuously recorded on a chart in a recorder via a polygraph system connected to said instrument.

The invention further provides a method of selecting a pharmaceutical agent for preventing or treating erectile dysfunction by using the method described above.

It relates to a method of preventing or treating erectile dysfunction in man, which comprises administering a pharmacologically effective amount of the compound selected by using the method described above or a pharmacologically acceptable salt thereof, for example, applied to a person suffering from erectile dysfunction, and use of the compound selected by using the method described above or a pharmacologically acceptable salt thereof for manufacturing a medicament for the prophylactic or therapeutic treatment of erectile dysfunction in man.

The selected compound of the invention may be a vasodilator agent or a phosphodiesterase 5 inhibitor.

### Detailed Description of the Preferred Embodiment

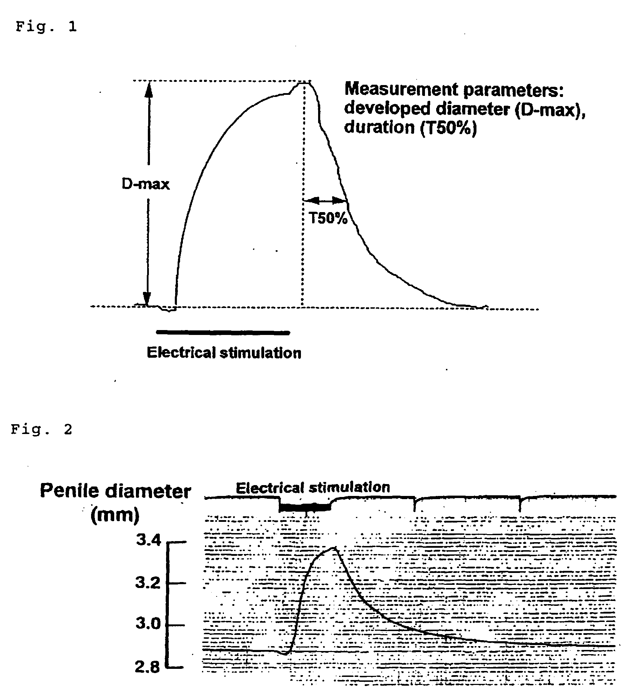

Fig. 1 is a graph for explanation of erectile response parameters used in evaluation of the effects of a drug. As the erectile response parameters, the maximal developed penile diameter (D-max, µm) induced by electrical stimulation, and the time (T50%, seconds) having elapsed from the maximum response to 50% recovery of the maximum response were used. And, Fig. 1 schematically shows a change in penile diameter induced by electrical stimulation of the cavernous nerve, obtained in this measurement method. The maximum increase of penile diameter is expressed as D-max (µm), and the time having elapsed from the maximum increase to 50% recovery of the maximum response is expressed as T50% (seconds). This measurement method can be used to judge the effect of a treated drug by comparing e.g. T50% (seconds) before and after administration of the drug.
Fig. 2 shows a record of the erectile response induced by electrical stimulation of the cavernous nerve and a representatively tracing of the penile diameter increase induced by electrical stimulation of the cavernous nerve.
Fig. 3 shows the effect of intravenously infused zaprinast on the recovery time (T50%) after the increase in the penile diameter induced by electrical stimulation of the cavernous nerve in anesthetized rats.

Four sequential 30-minute intravenous infusions of zaprinast (10, 30, 100 and 300 µg/kg/min, n = 6, filled circles) was administered to rats. The effect of zaprinast on the recovery time (T50%; the time having elapsed from the maximum response to 50% recovery of the maximum response) was compared with that of vehicle (5% glucose solution, n = 6, unfilled circles).

All values are the mean±standard error. *P<0.05 vs. 5% glucose solution.

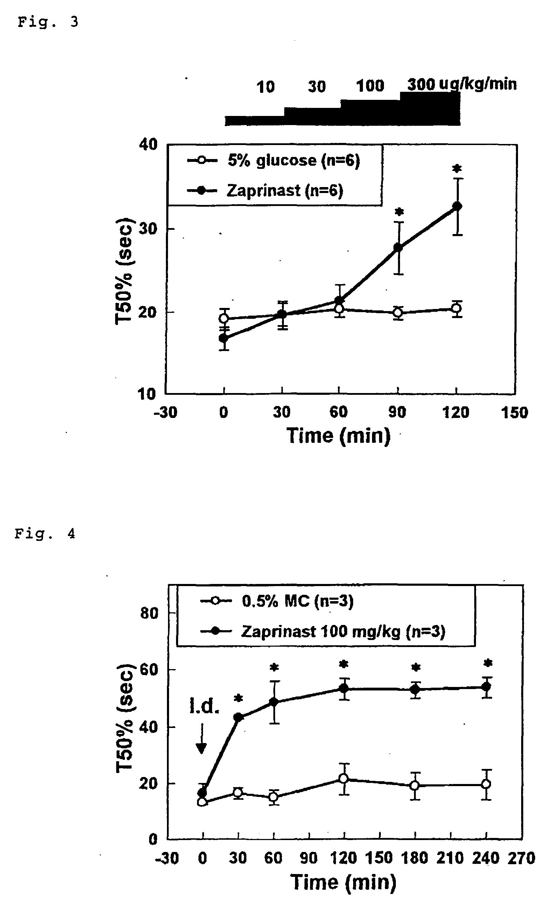

Fig. 4 shows the effect of intraduodenally administered zaprinast on the recovery time (T50%) after the increase in the penile diameter induced by electrical stimulation of the cavernous nerve in anesthetized rats.

Zaprinast (100 mg/kg, n = 3, filled circles) was intraduodenally (I.d.) administered to rats. The effect of zaprinast on the recovery time (T50%; the time having elapsed from the maximum response to 50% recovery of the maximum response) was compared with that of vehicle (0.5% methylcellulose solution, n = 3, unfilled circles).

All values are the mean±standard error. *P<0.05 vs. 0.5% methylcellulose solution.

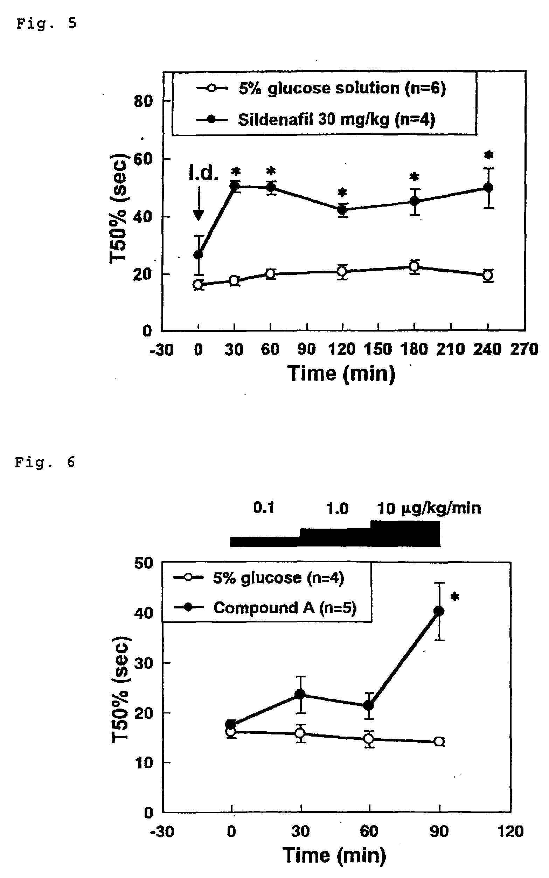

Fig. 5 shows the effect of intraduodenally administered sildenafil on the recovery time (T50%) after the increase in the penile diameter induced by electrical stimulation of the cavernous nerve in anesthetized rats.

Sildenafil (30 mg/kg, n = 4, filled circles) was intraduodenally (I.d.) administered to rats. The effect of sildenafil on the recovery time (T50%; the time having elapsed from the maximum response to 50% recovery of the maximum response) was compared with that of vehicle (0.5% glucose solution, n = 6, unfilled circles).

All values are the mean±standard error. *P<0.05 vs. 5% glucose solution.

Fig. 6 shows the effect of intravenously infused compound A on the recovery time (T50%) after the increase in the penile diameter induced by electrical stimulation of the cavernous nerve in anesthetized rats.

Three sequential 30-minute intravenous infusions of compound A (0.1, 1.0 and 10 µg/kg/min, n = 5, filled circles) was administered to rats. The effect of compound A on the recovery time (T50%; the time having elapsed from the maximum response to 50% recovery of the maximum response) was compared with that of vehicle (5% glucose solution, n = 4, unfilled circles).

All values are the mean±standard error. *P<0.05 vs. 5% glucose solution.

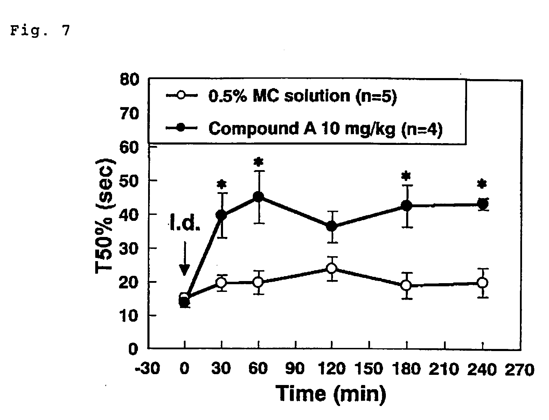

Fig. 7 shows the effect of intraduodenally administered compound A on the recovery time (T50%) after the increase in the penile diameter induced by electrical stimulation of the cavernous nerve in anesthetized rats.

Compound A (10 mg/kg, n = 4, filled circles) was intraduodenally (I.d.) administered to rats. The effect of compound A on the recovery time (T50%; the time having elapsed from the maximum response to 50% recovery of the maximum response) was compared with that of vehicle (0.5% methylcellulose solution, n = 5, unfilled circles).

All values are the mean±standard error. *P<0.05 vs. 0.5% methylcellulose solution.

The piezoelectric crystals used in the present invention are sensors of 2 mm or less in diameter having a frequency of 5 to 20 MHz, prepared from e.g. a ceramic oscillator of 5, 7, 10 and 20 MHz. The frequency of the ultrasonic wave is selected as necessary depending on animals or tissues used.

This measurement method can be used in measurement for a long time (at least 4 hours) since the corpus cavernosum in a penis are not damaged, no bleeding is caused, and repeated administration of heparin-containing physiological saline through a measurement tube is not necessary. It is also possible to continuously monitor penile diameter of an animal not only under anesthesia but also in an awaked state after recovery from operation conducted for fitting a measurement instrument.

The route of administration of a drug (compound) evaluated in this measurement method includes oral administration, intraduodenal administration, intravenous injection, intravenously infused injection, insertion into the urethra, application onto the penis, and direct injection into the penis.

This measurement method can be used to evaluate not only PDE 5 inhibitors but also drugs to directly dilate arteries, veins and cavernosal smooth muscles, such as PDE 3 inhibitors, nitrite compounds, α-blockers, potassium channel openers and prostaglandin E1 derivatives.

### Examples

Hereinafter, experimental examples are described to facilitate the understanding of the present invention, but the present invention is not limited by these examples. The electrical stimulation of the cavernous nerve is not necessary for evaluating drugs other than PDE 5 inhibitors.

The following literatures disclose processes for producing the compounds used in the Examples below: J. Med. Chem., 18, 1117-1122 (1975) for zaprinast in Examples 2 and 3; EP 0463756-A1 for sildenafil in Example 4; and JP-A 8-225541 for 4-[(3-chloro-4-methoxyphenethylamino]-1-(4-hydroxypiperidino)-6-phthaladinecarbonitrile hydrochloride (compound A) in Examples 5 and 6.

### Example 1: Measurement of the diameter of an erected penis induced by electrical stimulation of the cavernous nerve

Male Sprague Dawley rats weighing 350 to 450 g were allowed to fast for 1 day, then anesthetized by intraperitoneal administration of pentobarbital sodium (50 mg/kg), and placed in a supine position on a heat-insulating pad. To secure spontaneous respiration, a cannula was fitted into the respiratory tract. The operation was carried out according to the method of Quinlan et al. (J. Urol., 141, 656-661, 1989). A midline incision was made from umbilicus to pubis. The rat penis was denuded of skin, the prepuce was circumcised, and the testes were retracted. The scrotum was divided and packed into the upper abdomen along with loops of bowel. The right or left cavernous nerve was carefully isolated and hooked with a bipolar platinum electrode (MS-001, Unique Medical, Tokyo, Japan) connected to an electrical stimulator (SEN-7103, Nihon Kohden, Tokyo, Japan).

Then, a pair of 10 MHz piezoelectric crystals (about 1.5 mm × about 1.5 mm, Murata Mfg., Kyoto, Japan) were glued to the opposite surfaces of the adventitia of the penile erectile chamber (the shaft of the corpus cavernosum penis) using a medical adhesive agent (Aron Alpha, Sankyo, Tokyo, Japan). The penile diameter was measured continuously with an ultrasonic dimension system (UDM-5C, MECC, Fukuoka, Japan).

Unilateral electrical stimulation of the cavernous nerve was undertaken for 30 seconds using a square wave stimulator. The electrical stimulation voltage was 10 V. The parameters of electrical stimulation were a frequency of 20 Hz and duration of 2 m second. The induced erectile response is shown in Fig. 2.

### Example 2: Measurement of penile diameter increase during intravenous infusions of zaprinast (PDE 5 inhibitor) Preparation of an animal model

Male Sprague Dawley rats weighing 350 to 450 g were allowed to fast for 1 day, then anesthetized by intraperitoneal administration of pentobarbital sodium (50 mg/kg), and placed in a supine position on a heat-insulating pad. To secure spontaneous respiration, a cannula was fitted into the respiratory tract. The skin of the cervical region was incised, and a polyethylene tube (Becton Dickinson, Sparks, MD, USA) was inserted into the right jugular vein for intravenous infusion of vehicle or chemical.

The operation for electrical stimulation of the cavernous nerve and measurement of penile diameter were conducted in the same manner as in Example 1.

After the operation, it was confirmed that the penile diameter recorded on a chart in a recorder became stable, and then the erectile response was induced by cavernous nerve stimulation and recorded on the chart to obtain the initial value of the erectile response. Then, the intravenous infusion of vehicle or the intravenous infusions of zaprinast from a low dose at 30-minute intervals was conducted. For administration of vehicle, intravenous infusion for 120 minutes in total was conducted. The induction of erectile response by cavernous nerve stimulation was conducted for 25 to 30 minutes after administration of each dose was initiated. When the finding of suggesting awakening of the animal was observed during the experiment, a solution of pentobarbital sodium (about 0.2 ml) prepared by 10-fold diluting a stock solution was additionally administered intravenously into rats.

The doses of zaprinast were 10, 30, 100 and 300 µg/kg/min. A solution of the sample at each concentration was introduced into a syringe (10 ml), and the dose was increased from the lower dose at 30-minute intervals by an infusion pump (Model 210, Stoelting, Wood Dale, IL, USA). Zaprinast was dissolved in a 1 : 49 mixture of 1 N sodium hydroxide and 5% glucose solution. As the control, 5% glucose solution was used. The rate of the intravenous infusion was 1 ml/30 min.

The results are shown in Fig. 3.

The penile diameter before the first cavernous nerve stimulation was 2.812±0.050 mm (n = 6) in the group given zaprinast and 2.799±0.091 mm (n = 6) in the group given 5% glucose solution. The initial value of D-max induced by cavernous nerve stimulation was 530±48 µm in the group given zaprinast and 479±49 µm in the group given the glucose solution, and there was no statistically significant difference between the two groups. Further, D-max did not show any dose-dependent change during the intravenous infusion of zaprinast.

There was no significant difference between the two groups in the initial value of T50% as shown in Fig. 3. Zaprinast at doses of 100 and 300 µg/kg/min prolonged T50% in a dose-depending manner. T50% was 27.7±3.1 seconds (n = 6, P<0.05) in 100 µg/kg/min and 32.6±3.3 seconds (n = 6, P<0.05) in 300 µg/kg/min, which were significantly longer than their corresponding values (19.8±0.8 seconds and 20.4±1.0 seconds, respectively) in the vehicle group.

### Example 3: Measurement of penile diameter increase after an intraduodenal administration of zaprinast (PDE 5 inhibitor)

Male Sprague Dawley rats weighing 350 to 450 g were allowed to fast for 1 day, then anesthetized by intraperitoneal administration of pentobarbital sodium (50 mg/kg), and placed in a supine position on a heat-insulating pad. To secure spontaneous respiration, a cannula was fitted into the respiratory tract. The skin of the cervical region was incised, and a polyethylene tube (Becton Dickinson, Sparks, MD, USA) was inserted into the right jugular vein for additional intravenous injections of pentobarbital sodium. Further, the abdomen was opened little to insert a catheter into the duodenum, and zaprinast was administered through the catheter.

The operation for electrical stimulation of the cavernous nerve and measurement of penile diameter were conducted in the same manner as in Example 1.

After the operation, it was confirmed that the penile diameter recorded on a chart in a recorder became stable, and then the erectile response was induced by electrical stimulation of the cavernous nerve and recorded on the chart to obtain the initial value of the erectile response. Then, zaprinast at a dose of 100 mg/kg or its medium was administered into the duodenum. Zaprinast was previously suspended in 0.5% methylcellulose solution. The volume of the medium or the sample administered was 1 ml every 250 g body weight. At 30, 60, 120, 180 and 240 minutes after administration of zaprinast or vehicle, the erectile response was induced by cavernous nerve simulation.

The results are shown in Fig. 4.

The penile diameter before the first cavernous nerve simulation was 2.881±0.137 mm (n = 3) in the group given zaprinast and 2.825±0.047 mm (n = 3) in the group given 0.5 % methylcellulose solution as the medium. The initial value of D-max induced by cavernous nerve simulation was 502±47 µm in the zaprinast group and 479±94 µm in the vehicle group, and there was no statistically significant difference between the two groups.

As shown in Fig. 4, there was no significant difference in the initial value of T50% before administration between the zaprinast group (16.2±3.6 seconds, n = 3) and the vehicle group (12.9±0.6 seconds, n = 3). However, T50% was significantly prolonged (43.0±0.3 seconds, P<0.05) from 30 minutes after administration of 100 mg/kg zaprinast, and this action continued for 4 hours or more after the administration. Example 4: Measurement of penile diameter increase after an intraduodenal administration of sildenafil (PDE 5 inhibitor)

The erectile effect of intraduodenal administration of sildenafil (30 mg/kg) was evaluated in the same manner as in Example 3. Sildenafil was administered after dissolved in 5% glucose solution containing 5 mM phosphate (that is, a 1 : 199 mixture of 1 M phosphate and 5% glucose solution).

The results are shown in Fig. 5.

The penile diameter before the first cavernous nerve simulation was 3.229±0.091 mm (n = 4) in the sildenafil group and 3.250±0.238 mm (n = 6) in the vehicle group. The initial value of D-max induced by cavernous nerve simulation was 344±26 µm in the sildenafil group and 339±12 µm in the vehicle group, and there was no statistically significant difference between the two groups.

As shown in Fig. 5, there was no significant difference in the initial value of T50% before administration between in the sildenafil group (26.5±6.8 seconds, n = 4)) and the vehicle group (16.1±1.7 seconds, n = 6). However, T50% was significantly prolonged (50.5±2.0 seconds, P<0.05) from 30 minutes after administration of 30 mg/kg sildenafil, and this action continued for 4 hours or more after the administration. Example 5: Measurement of penile diameter increase during intravenous infusions of compound A (PDE 5 inhibitor)

The erectile effect of intravenous infusions of compound A was evaluated in the same manner as in Example 2. Compound A was administered after dissolved in a 1 : 199 mixture of 1 M phosphate and 5% glucose injection. The rate of the intravenous infusion was 1 ml/30 min.

The results are shown in Fig. 6.

The penile diameter before the first cavernous nerve simulation was 3.234±0.147 mm (n = 5) in the compound A group and 3.388±0.118 mm (n = 4) in the vehicle group. The initial value of D-max induced by cavernous nerve stimulation was 364±17 µm in the compound A group and 423±89 µm in the vehicle group, and there was no statistically significant difference between the two groups. Further, D-max did not show any dose-dependent change during intravenous infusions of compound A.

As shown in Fig. 6, there was no significant difference in the initial value of T50% before administration between the compound A group and the vehicle group. However, T50% during an intravenous infusion of compound A at a dose of 10 µg/kg/min was 40.2±5.7 seconds (n = 5, P<0.05), which was significantly longer than its corresponding value (14.1±0.8 seconds, n = 4) in the vehicle group.

### Example 6: Measurement of penile diameter increase after an intraduodenal administration of compound A

The erectile effect of an intraduodenal administration of compound A (10 mg/kg) was evaluated in the same manner as in Example 3. Compound A was administered after suspended in 0.5% methylcellulose solution. The volume of the medium or the sample administered was 1 ml every 250 g body weight.

The results are shown in Fig. 7.

The penile diameter before the first cavernous nerve stimulation was 3.419±0.277 mm (n = 4) in the compound A group and 2.805±0.042 mm (n = 5) in the vehicle group. The initial value of D-max induced by cavernous nerve stimulation was 402±15 µm in the compound A group and 504±54 µm in the vehicle group, and there was no statistically significant difference between the two groups.

As shown in Fig. 7, there was no significant difference in the initial value of T50% before administration between the compound A group (13.8±1.4 seconds, n = 4) and the vehicle group (15.1±1.5 seconds, n = 5). However, T50% was significantly prolonged (39.8±6.6 seconds, P<0.05) from 30 minutes after administration of 10mg/kg compound A, and this action continued for 4 hours or more after the administration.

The values of T50% in Figs. 3 to 7 are expressed as mean±standard error. The repeated measures analysis of variance concerning the erectile response parameters (D-max, T50 %) was conducted between the group given the medium and the group given the chemical or drug, and if there is a significant difference between the two groups, Student's unpaired t-test was conducted at each point in time. The level of significance was 5% on both sides.

As described above, the method for measurement of penile diameter by use of piezoelectric crystals according to the present invention is useful as a method of evaluating the efficacy of a therapeutic agent for male erectile dysfunction.

## Claims

1. A method of measuring a penile diameter of an animal by use of piezoelectric crystals.

2. The method according to claim 1, wherein the animal is dog, rabbit, rat or mouse.

3. The method according to claim 1, wherein signals from a pair of piezoelectric crystals fitted on the opposite surfaces of the adventitia of the penile erectile chamber are received by an ultrasonic dimension system, and the diameter of the animal's penis is continuously recorded on a chart in a recorder via a polygraph system connected to said instrument.

4. Use of the method according to claim 1 or 3, which is conducted with piezoelectric crystals to evaluate the effects of a compound on penile erection administered to the animal.

5. The use according to claim 4, wherein the compound is a vasodilator agent.

6. The use according to claim 4, wherein the compound is a phosphodiesterase 5 inhibitor.

7. A method of selecting a pharmaceutical agent for preventing or treating erectile dysfunction according to claim 4.

8. Use of the compound selected according to claim 4 or a pharmacologically acceptable salt thereof for manufacturing a medicament for the prophylactic or therapeutic treatment of erectile dysfunction in man.
